# EUROPÄISCHE PATENTSCHRIFT

(11) **EP 4 126 299 B1**
(45) Veröffentlichungstag und Bekanntmachung des Hinweises auf die Patenterteilung: **24.09.2025**
(21) Anmeldenummer: 21714128.2
(22) Anmeldetag: 19.03.2021
(51) Int. Cl.: B01D 46/00, F04B 39/16, F04B 53/20, A61L 9/20

(54) **FILTEREINHEIT FÜR EINEN KOMPRESSOR**
FILTER UNIT FOR A COMPRESSOR
UNITÉ DE FILTRATION POUR UN COMPRESSEUR

(30) Priorität: 24.03.2020 DE 102020203755
(43) Veröffentlichungstag der Anmeldung: 08.02.2023
(73) Patentinhaber: Bauer Kompressoren GmbH, 81477 München (DE)
(72) Erfinder: KAMPFL, Robert, 81379 München (DE); HUBER, Johannes, 86316 Friedberg (DE)
(74) Vertreter: Weickmann & Weickmann PartmbB
(86) Internationale Anmeldenummer: PCT/EP2021/057107
(87) Internationale Veröffentlichungsnummer: WO 2021/191081

(56) Entgegenhaltungen:
- WO-A2-99/22610
- CN-U- 209 838 649
- JP-A- H0 223 278
- JP-U- 3 009 261

## Beschreibung

Die vorliegende Erfindung betrifft einen mit einer Filtereinheit ausgestatteten Kompressor sowie ein Verfahren zum Komprimieren und Abfüllen von Atemluft mittels eines derartigen Kompressors.

Zwar bestehen beim Komprimieren und Abfüllen von Gasen und insbesondere Atemluft bereits strenge Vorschriften hinsichtlich der Güte und maximal zulässigen Verschmutzungen, wie beispielsweise die Atemluftnorm EN12021:2014-07, gemäß welcher alle Verunreinigungen weniger als ein Zehntel der nationalen 8-Stunden-Expositionsgrenzen betragen müssen, es ist jedoch gerade in jüngster Zeit auch der Wunsch bzw. die Notwendigkeit aufgekommen, auch möglicherweise in dem zu komprimierenden Gas vorliegende Viren vor dem Komprimieren und Abfüllen davon zu inaktivieren. Es versteht sich somit, dass im Kontext der vorliegenden Beschreibung der Begriff "Gas" stets auch Gasgemische umfassen soll.

Eine derartige Inaktivierung von Viren ist aufgrund ihrer Struktur und Größe nach aktuellem Erkenntnisstand nur mittels Hitze, Druck, geeigneten Chemikalien und UV-Strahlung möglich.

Bei Verwendung von Hitze ist eine Verweilzeit von 8 Minuten bei über 120°C erforderlich. Zwar könnten derartige Bedingungen im Ansaugbereich eines Kompressors geschaffen werden, das zu komprimierende Gas müsste anschließend allerdings aufwendig wieder gekühlt werden, da übliche Kompressoren eine maximale Ansaugtemperatur von etwa 45°C aufweisen, welche nicht überschritten werden darf.

Die Temperatur im Kompressor selbst kann andererseits zwar teilweise über 200°C betragen, allerdings liegt die Verweilzeit des Gases in diesem Zustand in der Regel bei lediglich einigen Sekunden, so dass eine zuverlässige Inaktivierung von Viren hierdurch nicht gegeben ist. Außerdem ist zu bedenken, dass ein Aufheizen von bereits unter Druck stehenden Behältern eine zusätzliche deutliche Druckerhöhung mit sich bringt, so dass der Behälter hierdurch zerstört werden könnte oder auf einen höheren Druck ausgelegt werden müsste, was zu einer deutlichen Kostensteigerung führen würde.

Zuletzt würde eine Temperaturerhöhung vor dem Befüllen des Behälters dazu führen, dass nach Abkühlung des Gases der Druck in dem zu befüllenden Behälter wesentlich abfällt und die vorgesehene Gasmenge daher nicht gespeichert werden kann. Somit besteht keine Möglichkeit, eine ausreichende Erhitzung des abzufüllenden Gases zu irgendeinem Zeitpunkt des Kompressions- und Abfüllvorgangs in einer effizienten und wirtschaftlich sinnvollen Weise durchzuführen.

In ähnlicher Weise ist auch eine Inaktivierung von Viren mittels Druck oder Chemikalien nicht umsetzbar, da einerseits Drücke von über 4000 bar notwendig wären, die ebenfalls nicht in einer wirtschaftlich sinnvollen Weise realisiert werden können und andererseits der Einsatz von viruziden Desinfektionsmitteln für viele Prozesse, insbesondere den Fall eines Komprimierens und Abfüllens von Atemluft, ausscheidet.

Hierbei ist aus der JP H02 23278 A eine Filtereinheit für einen Kompressor bekannt, welche ein Gehäuse, einen Vorfilter, eine Ultraviolett-Bestrahlungseinheit und ein Vorschaltgerät zur Ansteuerung der Bestrahlungseinheit umfasst. Der Vollständigkeit halber sei ferner auf die CN 209 839 649 U verwiesen, sowie auf die JP 3 009261 U, welche sich konkret mit einer Beschaltung einer Kompressor-Vorrichtung befasst, einschließlich ihres Motors und einer UV-Lampe

Es ist somit die Aufgabe der vorliegenden Erfindung, einen Kompressor bereitzustellen, mit welchem in einer zuverlässigen und effizienten Weise in dem zu komprimierenden Gas enthaltene Viren inaktiviert werden können.

Zu diesem Zweck umfasst die Filtereinheit für den erfindungsgemäßen Kompressor ein Gehäuse mit einem Einlass und einem Auslass für zu komprimierendes Gas, einen in Strömungsrichtung des zu komprimierenden Gases stromabwärts des Einlasses angeordneten Partikel-Vorfilter, eine in Strömungsrichtung des zu komprimierenden Gases stromabwärts des Partikel-Vorfilters angeordnete Ultraviolett-Bestrahlungseinheit, welche dazu eingerichtet ist, ultraviolettes Licht abzustrahlen, und ein Vorschaltgerät, welches dazu eingerichtet und angeordnet ist, die Ultraviolett-Bestrahlungseinheit anzusteuern. Hierbei ist die Filtereinheit derart ausgebildet, dass an dem Einlass zugeführtes zu komprimierendes Gas zunächst den Partikel-Vorfilter durchläuft, anschließend von der Ultraviolett-Bestrahlungseinheit bestrahlt wird und schließlich durch den Auslass dem Kompressor zuführbar ist.

Es ist demzufolge der Verdienst der vorliegenden Erfinder, erkannt zu haben, dass durch die Filtereinheit bereits vor der eigentlichen Kompression des Gases dieses von aktiven Viren befreit werden kann, wobei der Partikel-Vorfilter die Ultraviolett-Bestrahlungseinheit vor Verschmutzungen und einer damit einhergehenden Verschlechterung ihres Wirkungsgrads und der Inaktivierungsrate schützt.

Das Vorschaltgerät ist wiederum für die Versorgung der Bestrahlungseinheit mit elektrischer Energie verantwortlich und kann beispielsweise je nach Ausführung davon dazu eingerichtet sein, einen erhöhten Strom während eines Einschaltvorgangs zu liefern und anschließend einen im wesentlichen konstanten Strom.

Weiterhin ist die hier beschriebene Filtereinheit sowohl für mobile als auch stationäre Kompressoranlagen geeignet, da ihre Installation im Ansaugstrang im atmosphärischen Bereich erfolgt. Hierdurch ist auch eine besondere Eignung der erfindungsgemäßen Einheit als Nachrüstlösung gegeben, da diese vollständig autark betrieben werden kann und lediglich eine eigene Stromversorgung für das Vorschaltgerät und die Bestrahlungseinheit benötigt. Weiterhin sei festgehalten, dass wenngleich in dieser Beschreibung in erster Linie auf die Inaktivierung von Viren in dem zu komprimierenden Gas abgezielt wird, so doch mit der erfindungsgemäßen Filtereinheit ebenfalls Bakterien, Sporen, etc. wirksam bekämpft werden können.

In einer vorteilhaften Ausführungsform kann das Gehäuse der Filtereinheit im Bereich der Ultraviolett-Bestrahlungseinheit wenigstens abschnittsweise, vorzugsweise vollständig, insbesondere an seiner Innenseite, aus eloxiertem Aluminium gefertigt sein. Im Gegensatz zu beispielsweise Edelstahl weist eloxiertes Aluminium nämlich im relevanten Ultraviolett-Wellenlängenbereich hervorragende Reflexionseigenschaften auf, durch welche die Effizienz der Bestrahlung erhöht wird, während es durch die Absorption der Strahlung in Edelstahl ferner zu einer unerwünschten verstärkten Aufheizung des Gehäuses kommen könnte. Insbesondere könnte das Gehäuse wenigstens teilweise aus einem Typ von Aluminiumrohr gefertigt, welcher üblicherweise bei der Patronenfertigung für Gase in großen Stückzahlen verwendet wird. Eine kostengünstige Versorgung mit dieser kritischen Komponente ist demnach sichergestellt, wobei insbesondere Patronenrohre mit den Artikelnummern 61089, 62333 und 60174 zum Einsatz kommen könnten.

Ferner kann das Gehäuse im Bereich der Ultraviolett-Bestrahlungseinheit länglich ausgebildet sein, insbesondere zylindrisch, beispielsweise kreiszylindrisch. Hierdurch wird nicht nur ein geeigneter Aufnahmeraum für die üblicherweise ebenfalls längliche Bestrahlungseinheit geschaffen, sondern es wird auch die Verweilzeit des Gases im effektiven Bestrahlungsbereich erhöht und somit die Effizienz der Filtereinheit sichergestellt. Des Weiteren wird durch eine kreiszylindrische Form des Gehäuses im Bereich der Ultraviolett-Bestrahlungseinheit dafür gesorgt, dass ein laminarer Gasstrom entlang der Bestrahlungseinheit erzeugt wird, in welchem das Gas stets in einem wohldefinierten Abstand daran vorbeigeführt wird. Außerdem werden nachteilhafte Strömungseigenschaften innerhalb des Gehäuses vermieden, die durch Hinterschneidungen oder ähnliches erzeugt werden könnten.

Eine weitere Maßnahme zur Erhöhung der Effizienz der Einheit kann darin bestehen, das Vorschaltgerät ferner dazu einzurichten, die Ansteuerung der Ultraviolett-Bestrahlungseinheit an eine Umgebungstemperatur und/oder eine Temperatur des zu komprimierenden Gases anzupassen, welche durch einen Benutzer mithilfe einer Eingabeeinheit eingebbar oder mittels einer geeignet angeordneten Sensoreinheit erfassbar ist. Diese Weiterbildung beruht auf der Tatsache, dass die Inaktivierungsrate üblicherweise eine Temperaturabhängigkeit aufweist, so dass die Ultraviolett-Bestrahlungseinheit wenigstens so leistungsstark ausgelegt sein muss, um am wenigstens effektiven Temperaturbereich dennoch die gewünschte Inaktivierungsrate zu erzielen. Beispielsweise könnte sie bei höheren Temperaturen hinsichtlich ihrer Strahlungsleistung durch das Vorschaltgerät heruntergeregelt werden. Die verwendete Eingabeeinheit und/oder die verwendete Sensoreinheit können von beliebigen geeigneten bekannten Typen solcher Einheiten und entsprechend angeordnet sowie mit dem Vorschaltgerät betriebsmäßig gekoppelt sein.

Als Sicherheitsmaßnahme und um zu verhindern, dass bei einem Ausfall oder einem anderen Problem mit der Ultraviolett-Bestrahlungseinheit nicht ausreichend behandeltes Gas komprimiert und/oder abgefüllt wird, kann die erfindungsgemäße Filtereinheit ferner eine Überwachungseinheit umfassen, welche dazu eingerichtet ist, einen korrekten Betrieb der Ultraviolett-Bestrahlungseinheit zu überwachen. Hierbei kann unter anderem daran gedacht werden, mit geeigneten Mitteln die elektrischen Eigenschaften der Ultraviolett-Bestrahlungseinheit zu überwachen, also beispielsweise eine Unterbrechung des Versorgungsstromkreises, einen Ausfall der Bestrahlungseinheit während des Betriebs, einen Kurzschluss oder einen Bruch von Strahlerelektroden, ein Unterschreiten der minimal zulässigen Netzspannung oder ein Überschreiten der maximalen an der Bestrahlungseinheit anliegenden Spannung. Weiterhin könnte auch ein Sensor für ultraviolette Strahlung innerhalb des Gehäuses vorgesehen werden, der bei einer unter einem vorgegebenen Schwellenwert liegenden Strahlungsintensität ein entsprechendes Signal ausgibt.

Eine Möglichkeit, einen von der Überwachungseinheit festgestellten inkorrekten Betrieb der Ultraviolett-Bestrahlungseinheit weiterzuverarbeiten, kann darin bestehen, dass die Filtereinheit ferner eine Benachrichtigungseinheit umfasst, welche mit der Überwachungseinheit betriebsmäßig gekoppelt und dazu eingerichtet ist, bei einem Feststellen einer Abweichung von dem korrekten Betrieb der Ultraviolett-Bestrahlungseinheit eine Benachrichtigung auszugeben. Hierbei kann sowohl an eine Anzeigevorrichtung wie ein Display oder auch nur eine Warnleute gedacht werden oder alternativ oder zusätzlich an eine akustische Signaleinheit. Zudem kann neben der Benachrichtigungseinheit auch eine Datenausgabe der Überwachungseinheit an eine andere Vorrichtung vorgesehen werden, beispielsweise an eine weiter unten noch diskutierte Steuereinheit des entsprechenden Kompressors.

Da übliche Kompressoren in ihrem Betrieb beträchtliche Vibrationen erzeugen, die insbesondere in Ausführungsformen, in denen die Filtereinheit direkt in Kontakt mit dem Kompressor steht, über kurz oder lang zu Beschädigungen der Ultraviolett-Bestrahlungseinheit führen können, kann es vorteilhaft sein, wenn die Ultraviolett-Bestrahlungseinheit innerhalb des Gehäuses in einer vibrationsgedämpften Weise angebracht ist, insbesondere mittels wenigstens einer Federklemme, welche vorzugsweise wenigstens abschnittsweise aus einem Keramik- und/oder Blechmaterial gefertigt ist.

Hierbei sind Materialien wie Keramik und Metallblech kostengünstigeren Alternativen aus Kunststoff aufgrund ihrer größeren Beständigkeit gegenüber ultravioletter Strahlung vorzuziehen.

Alternativ oder zusätzlich kann insbesondere in Ausführungsformen, in welchen die erfindungsgemäße Filtereinheit direkt an dem Gehäuse des betreffenden Kompressors angeordnet werden soll, diese wenigstens eine an der Außenseite des Gehäuses vorgesehene Halterung umfassen, welche vorzugsweise wenigstens ein Dämpfungselement für mechanische Vibrationen umfasst, beispielsweise ein Elastomer-Dämpfungselement, wobei die Halterung vorzugsweise wenigstens einen Permanentmagneten umfasst, um ein einfaches und werkzeugloses Anbringen davon an Oberflächen aus Eisenmetallen zu ermöglichen. Alternativ könnten jedoch selbstverständlich auch andere Anbringungsmittel vorgesehen werden, beispielsweise Schrauben oder ein Haftmaterial.

Wenngleich Strahlung im gesamten ultravioletten Spektrum eine inaktivierende Wirkung auf Viren, Bakterien, etc. entfaltet, so kann die Ultraviolett-Bestrahlungseinheit der erfindungsgemäßen Filtereinheit insbesondere dazu eingerichtet ist, ultraviolettes Licht im UVC-Bereich abzustrahlen, vorzugsweise bei einer Wellenlänge von etwa 254 nm. **In** diesem Wellenlängenbereich abstrahlende Leuchtmittel sind kommerziell erhältlich und derartige Strahlung bietet gute Eigenschaften hinsichtlich ihrer Absorption in vielen Gasen und ihrer bereits angesprochenen Reflexion an eloxiertem Aluminium, wobei sie ferner auch nicht zu einer unerwünschten Erzeugung von Ozon aus Sauerstoff führt, die bei noch kurzwelligerer Strahlung auftreten würde.

Wenngleich der Partikel-Vorfilter von unterschiedlichsten geeigneten Typen sein kann, solange er ein Eindringen von Schmutzpartikeln in das Gehäuse der erfindungsgemäßen Filtereinheit zuverlässig verhindert, so zeigt sich, dass hierfür insbesondere Partikel-Vorfilter geeignet sind, welche wenigstens von der Filterklasse F7 sind.

Ein weiterer Beitrag zur Sicherstellung des vorgesehenen Betriebs der Filtereinheit kann darin bestehen, diese mit einer Unterdruck-Überwachungseinheit bereitzustellen, welche dazu eingerichtet ist, über eine übermäßige Druckdifferenz zwischen dem Innenraum und der Umgebung des Gehäuses zu benachrichtigen, und welche insbesondere im Bereich des Partikel-Vorfilters angeordnet sein kann. Hierbei kann an handelsübliche Unterdruckwächter gedacht werden, die beim Auftreten einer solchen Druckdifferenz, welche einen vorgegebenen Schwellenwert übersteigt, ein entsprechendes Signal ausgeben, welches darauf hinweist, dass der Partikel-Vorfilter durch über seine Betriebsdauer angesammelte Partikel verstopft ist und ersetzt werden muss.

Wie bereits angesprochen betrifft die vorliegende Erfindung einen Kompressor, insbesondere einen Atemluftkompressor und/oder mehrstufigen Kolbenkompressor, umfassend einen Kompressorblock mit wenigstens einem Kompressormotor und eine Filtereinheit. Mehrstufige Kolbenkompressoren sind dem Fachmann an sich bekannt und umfassen neben mehreren hintereinandergeschalteten Kolbenstufen üblicherweise auch noch Kühler-, Trocknungs- und Abscheidereinheiten zum Behandeln des verdichteten Gases.

Für den oben beschriebenen Fall, dass die verwendete Filtereinheit eine Überwachungseinheit umfasst und der Kompressor ferner eine Steuereinheit umfasst, so kann diese mit der Überwachungseinheit betriebsmäßig gekoppelt oder integriert und dazu eingerichtet sein, bei einem Feststellen einer Abweichung von dem korrekten Betrieb der Ultraviolett-Bestrahlungseinheit den Betrieb des Kompressors anzupassen, beispielsweise den Kompressor abzuschalten oder ein Spülventil zu öffnen. Auf diese Weise kann auf Seiten des Kompressors verhindert werden, dass nicht ausreichend behandeltes Gas abgefüllt wird, wobei durch die Verwendung eines Spülventils, welches zwar zulässt, dass der Kompressor das durch die Filtereinheit angesaugte Gas verdichtet, dieses jedoch statt es abzufüllen einfach aus dem Kompressor an die Umgebung abgibt, ein wiederholtes Ein- und Ausschaltung des wenigstens einen Kompressormotors verhindert werden kann, wenn nur ein temporäres Problem mit der Ultraviolett-Bestrahlungseinheit vorliegt.

Alternativ oder zusätzlich könnte der Kompressor auch eine Zeitnahmeeinheit umfassen, welche dazu eingerichtet ist, einen zeitlichen Abstand zwischen einer Inbetriebnahme der Ultraviolett-Bestrahlungseinheit und des wenigstens einen Kompressormotors sicherzustellen. Hierbei kann einerseits an einen festen Wert für eine solche Verzögerung gedacht werden, die die verwendete Bestrahlungseinheit erfahrungsgemäß benötigt, um ihre vorgesehene Leistung zu erreichen, oder auch an eine elektrische oder elektronische Schaltung, welche der Bestrahlungseinheit zugeordnet ist und ein Startsignal für den Kompressormotor erst freigibt, wenn die vorgesehene Leistung erreicht worden ist. In diesem Zusammenhang könnte als Inbetriebnahme des wenigstens einen Kompressormotors auch ein Schließen eines entsprechenden Spülventils verstanden werden. In jedem Fall wird durch diese Maßnahme sichergestellt, dass ein Abfüllen des komprimierten Gases erst begonnen wird, wenn die Ultraviolett-Bestrahlungseinheit ihren vorgesehenen Betriebszustand erreicht hat, also beispielsweise eine Aufwärmphase durchlaufen hat.

Wenngleich die Filtereinheit mit ihrem Auslass direkt an einen Gaseinlass des Kompressorblocks angeflanscht werden könnte, so kann ebenfalls ein Ansaugschlauch zwischen der Filtereinheit und dem Kompressorblock vorgesehen sein, welcher an dem Auslass der Filtereinheit vorzugsweise mittels elastischem Material abgedichtet angebracht ist. Auf diese Weise kann eine flexible Positionierung der Filtereinheit erzielt werden, beispielsweise kann die Filtereinheit über die oben bereits angesprochenen Permanentmagnete am Gehäuse des Kompressorblocks befestigt werden.

Der erfindungsgemäße Kompressor umfasst in jedem Fall eine Sicherheitseinrichtung, welche dazu eingerichtet ist, sicherzustellen, dass ein Betrieb des Kompressors nur möglich ist, wenn die Filtereinheit in Betrieb ist, beispielsweise auf Grundlage der oben beschriebenen Überwachungseinheit der Filtereinheit. Hierbei umfasst die Sicherheitseinrichtung zusätzlich Mittel, durch welche ein Betrieb des Kompressors ausnahmsweise auch in Fällen erlaubt werden kann, in welchen die Filtereinheit nicht in Betrieb ist, beispielsweise wenn in Katastrophenfällen die Verfügbarkeit der Grundfunktion des Kompressors die Nachteile der außer Betrieb stehenden Filtereinheit überwiegt. Als derartiges Mittel kann unter anderem ein Schlüsselschalter oder eine Eingabevorrichtung für einen Code dienen.

Zuletzt betrifft die vorliegende Erfindung ein Verfahren zum Komprimieren und Abfüllen von Atemluft mittels eines erfindungsgemäßen Kompressors, wobei die zu komprimierende Atemluft durch die Filtereinheit angesaugt, anschießend durch den wenigstens einen Kompressormotor verdichtet und schließlich bei einem Druck von beispielsweise zwischen 90 und 550 bar abgefüllt wird.

Weitere Merkmale und Vorteile der vorliegenden Erfindung werden aus der nachfolgenden Beschreibung einer Ausführungsform noch deutlicher, wenn diese zusammen mit den beiliegenden Figuren betrachtet wird. Diese zeigen im Einzelnen:
- Figur 1: eine Filtereinheit in schematischer Seitenansicht;
- Figur 2: die Filtereinheit aus Figur 1 in einer Schnittansicht entlang einer Schnittebene A-A aus Figur 1; und
- Figuren 3a und 3b: vergrößerte Detailansichten von zwei Bereichen aus Figur 2.

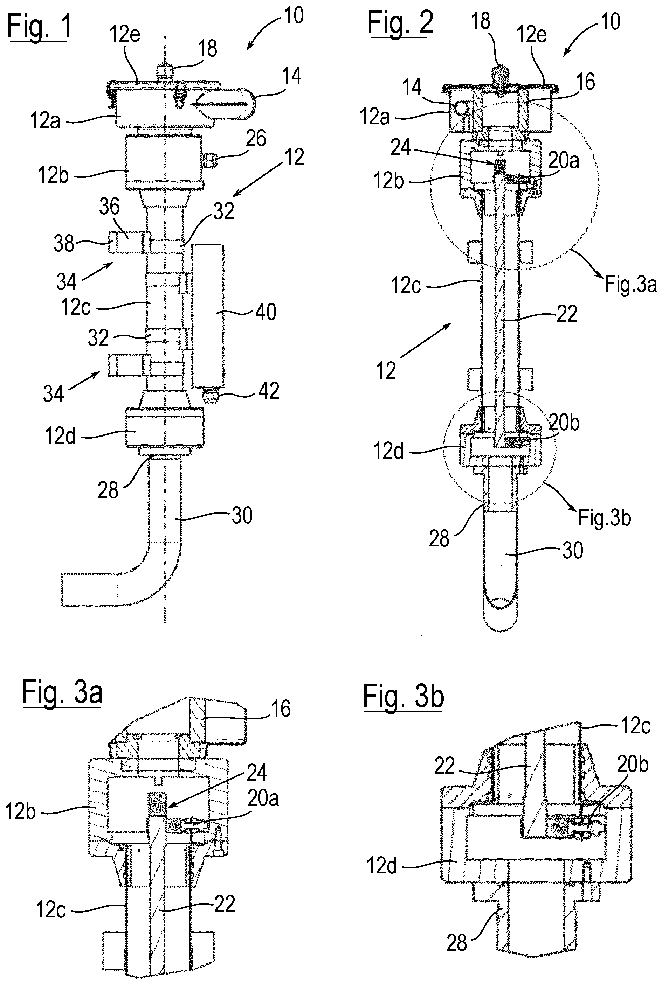

In den Figuren 1 und 2 ist jeweils eine Filtereinheit eines erfindungsgemäßen Kompressors gezeigt und ganz allgemein mit dem Bezugszeichen 10 bezeichnet. Die Filtereinheit 10 umfasst ein mehrteiliges Gehäuse 12, welches an einem ersten, in den Figuren obersten Teil 12a einen Einlass 14 für Gas umfasst, an welchen optional ein Ansaugschlauch angeschlossen werden kann. Die im Folgenden beschriebenen einzelnen Teile des mehrteiligen Gehäuses 12 sind jeweils fest miteinander verbunden, beispielsweise vernietet, verschraubt, verschweißt oder ähnliches, wobei in manchen Ausführungsformen auch mehrere der Teile des Gehäuses 12 miteinander einstückig ausgebildet sein können.

In diesem ersten Teil 12a des Gehäuses 12 ist in einer Kammer ein Filtermaterial aufgenommen, welches einen ringförmigen Partikel-Vorfilter 16 bildet, welcher von dem durch den Einlass 14 einströmenden Gas durchlaufen wird, bevor dieses in den eigentlichen Innenraum des Gehäuses 12 eintritt. Ebenfalls dem ersten Teil 12a des Gehäuses 12 zugeordnet umfasst die Filtereinheit 10 eine Unterdruck-Überwachungseinheit 18 zum Anzeigen einer möglichen Verstopfung des Partikel-Vorfilters 16. Indem der erste Teil 12a des Gehäuses 12 an seiner Oberseite durch eine lösbar aufgespannte und abgedichtete Kappe 12e verschlossen ist, kann an dieser Stelle ein öffenbarer Zugriff in den Innenraum des Gehäuses 12 geschaffen werden, durch welchen beispielsweise in regelmäßigen Abständen das Filtermaterial des Partikel-Vorfilters 16 entnommen und ersetzt werden kann.

Unterhalb des ersten Teils 12a schließt sich ein zweiter Teil 12b des Gehäuses 12 an, welcher in Figur 3a in einer vergrößerten Detailansicht erneut gezeigt ist und in welchem ein erstes Anbringungselement 20a für eine stabförmige Ultraviolett-Bestrahlungseinheit 22 angeordnet ist, welche eingesetzt und ausgetauscht werden kann, indem der mittels eines nicht dargestellten Gewindes auf den weiter unten beschriebenen dritten Teil 12c des Gehäuses 12 aufgeschraubte zweite Teil 12b davon abgeschraubt und entfernt wird. Die Bestrahlungseinheit 22 kann beispielsweise als Leuchtröhre für ultraviolettes Licht bei einer Wellenlänge von etwa 254 nm ausgebildet sein, durch welches in dem innerhalb des Gehäuses strömenden Gas eventuell vorhandene Viren, Bakterien, Sporen, etc. inaktiviert oder abgetötet werden können.

Das erste Anbringungselement 20a ist in der gezeigten Ausführungsform durch eine Federklemme gebildet, welche die Bestrahlungseinheit 22 in einer vibrationsgedämpften Weise haltert, wobei eine Fassung 24 zur elektrischen Versorgung der Bestrahlungseinheit 22 auf diese von oben her lösbar aufgesteckt ist. Des Weiteren findet sich an der Außenseite des zweiten Teils 12b des Gehäuses 12 eine Kabelverschraubung 26, an welcher ein Stromkabel zur Versorgung der Ultraviolett-Bestrahlungseinheit anbringbar ist, wobei ferner eine in den Figuren nicht zu sehende elektrische Verbindung zwischen der Kabelverschraubung 26 und der Fassung 24 innerhalb des zweiten Teils 12b des Gehäuses 12 vorgesehen ist.

Von dem ersten Anbringungselement 20a aus erstreckt sich die Bestrahlungseinheit 22 durch einen dritten Teil 12c des Gehäuses 12 bis zu einem vierten Teil 12d, in welchem sie in analoger Weise von einem zweiten Anbringungselement 20b gehaltert ist, was in Figur 3b ebenfalls in vergrößerter Detailansicht dargestellt ist. Hierbei ist der dritte Teil 12c des Gehäuses 12 aus eloxiertem Aluminium gebildet, da dieses Material ultraviolettes Licht der genannten Wellenlänge reflektiert und somit sowohl den Wirkungsgrad der Vorrichtung erhöht als auch einer übermäßigen Aufheizung davon entgegenwirkt. Die anderen Teile des Gehäuses 12 können ebenfalls aus Aluminium oder aber auch aus einem anderen Material, beispielsweise Edelstahl gebildet sein, da diese der Strahlung der Bestrahlungseinheit 22 wesentlich weniger ausgesetzt sind.

An der Unterseite des vierten Teils 12d des Gehäuses 12 ist schließlich ein flanschförmiger Auslass 28 angebracht, an welchem in der in den Figuren gezeigten Konfiguration ein Ansaugschlauch 30 angebracht ist, durch welchen das bestrahlte Gas einem hier nicht dargestellten Kompressorblock zugeführt werden kann. Es sei darauf hingewiesen, dass im Bereich des dritten und vierten Teils 12c, 12d des Gehäuses 12 sowie des Auslasses 28 keine weiteren Zugänge in den Innenraum des Gehäuses 12 vorgesehen sind, so dass an dieser Seite der Filtereinheit 10, an welcher das Gas bereits bestrahlt worden ist und somit in einem für eine Kompression und schließlich eine Abfüllung bereiten Zustand vorliegt, keine aufwendigen Maßnahmen zur Abdichtung des Gehäuses 12 getroffen werden müssen und gleichzeitig kritische Verunreinigungen des behandelten Gases zuverlässig ausgeschlossen werden.

Weiterhin sei drauf hingewiesen, dass an der Außenseite des Gehäuses 12, insbesondere im Bereich des dritten Teils 12c davon mittels Montageschellen 32 zwei Halterungen 34 montiert sind, welche jeweils ein Elastomer-Dämpfungselement 36 und einen Permanentmagneten 38 umfassen und mittels welchen die Filtereinrichtung 10 an einer Fläche aus einem Eisenmetall angebracht werden kann, beispielsweise einer Außenseite der nicht gezeigten Kompressoranlage. Gemeinsam mit den oben angesprochenen Anbringungselementen 20a und 20b dienen die Dämpfungselemente 36 zum Entkoppeln der Filtereinrichtung 10 und insbesondere der Ultraviolett-Bestrahlungseinheit 22 von den im Betrieb des Kompressorblocks auftretenden Vibrationen und Erschütterungen.

Weiterhin ist in ähnlicher Weise durch zwei Montageschellen 32 ein Vorschaltgerät 40 in Form eines Schaltkastens an dem Gehäuse 12 montiert, welches sämtliche zum Betrieb der Filtereinheit 10 und insbesondere der Ultraviolett-Bestrahlungseinheit notwendigen Steueraufgaben sowie über eine weitere Kabelverschraubung 42 und ein nicht dargestelltes Kabel die elektrische Versorgung davon übernimmt. Das Vorschaltgerät 40 selbst wird über einen nicht dargestellten Anschluss aus dem Netz gespeist, so dass die Filtereinheit 10 abgesehen hiervon autark betrieben werden kann. Das Vorschaltgerät 40 kann ferner mit optional vorzusehenden und hier nicht gezeigten weiteren elektronischen Komponenten, wie beispielsweise Sensoren für die Umgebungstemperatur oder die Temperatur des in die Filtereinheit 10 angesaugten Gases, einer Überwachungseinheit für die Funktion der Ultraviolett-Bestrahlungseinheit, einer Benachrichtigungseinheit für einen Benutzer und/oder einer Steuereinheit des Kompressorblocks betriebsmäßig gekoppelt sein.

## Patentansprüche

1. Kompressor, insbesondere Atemluftkompressor und/oder mehrstufiger Kolbenkompressor, umfassend einen Kompressorblock mit wenigstens einem Kompressormotor und eine Filtereinheit (10), die Filtereinheit (10) umfassend:
- ein Gehäuse (12) mit einem Einlass (14) und einem Auslass (28) für zu komprimierendes Gas;
- einen in Strömungsrichtung des zu komprimierenden Gases stromabwärts des Einlasses (14) angeordneten Partikel-Vorfilter (16);
- eine in Strömungsrichtung des zu komprimierenden Gases stromabwärts des Partikel-Vorfilters (16) angeordnete Ultraviolett-Bestrahlungseinheit (22), welche dazu eingerichtet ist, ultraviolettes Licht abzustrahlen; und
- ein Vorschaltgerät (40), welches dazu eingerichtet und angeordnet ist, die Ultraviolett-Bestrahlungseinheit (22) anzusteuern;
wobei die Filtereinheit (10) derart ausgebildet ist, dass an dem Einlass (14) zugeführtes zu komprimierendes Gas zunächst den Partikel-Vorfilter (16) durchläuft, anschließend von der Ultraviolett-Bestrahlungseinheit (22) bestrahlt wird und schließlich durch den Auslass (28) dem Kompressor zuführbar ist,
**dadurch gekennzeichnet, dass**
der Kompressor ferner eine Sicherheitseinrichtung umfasst, welche dazu eingerichtet ist, sicherzustellen, dass ein Betrieb des Kompressors nur möglich ist, wenn die Filtereinheit in Betrieb ist, und welche zusätzlich Mittel umfasst, durch welche ein Betrieb des Kompressors ausnahmsweise auch in Fällen erlaubt werden kann, in welchen die Filtereinheit nicht in Betrieb ist.

2. Kompressor nach Anspruch 1, wobei das Gehäuse (12) im Bereich der Ultraviolett-Bestrahlungseinheit (22) wenigstens abschnittsweise, vorzugsweise vollständig, insbesondere an seiner Innenseite, aus eloxiertem Aluminium gefertigt ist, wobei beispielsweise Patronenrohre, insbesondere mit den Artikelnummern 61089, 62333 und 60174 zum Einsatz kommen können.

3. Kompressor nach einem der vorhergehenden Ansprüche, wobei das Gehäuse (12) im Bereich der Ultraviolett-Bestrahlungseinheit (22) länglich ausgebildet ist, insbesondere zylindrisch, beispielsweise kreiszylindrisch.

4. Kompressor nach einem der vorhergehenden Ansprüche, wobei das Vorschaltgerät (40) ferner dazu eingerichtet ist, die Ansteuerung der Ultraviolett-Bestrahlungseinheit (22) an eine Umgebungstemperatur und/oder eine Temperatur des zu komprimierenden Gases anzupassen, welche durch einen Benutzer mithilfe einer Eingabeeinheit eingebbar oder mittels einer Sensoreinheit erfassbar ist.

5. Kompressor nach einem der vorhergehenden Ansprüche, ferner umfassend eine Überwachungseinheit, welche dazu eingerichtet ist, einen korrekten Betrieb der Ultraviolett-Bestrahlungseinheit (22) zu überwachen.

6. Kompressor nach Anspruch 5, ferner umfassend eine Benachrichtigungseinheit, welche mit der Überwachungseinheit betriebsmäßig gekoppelt und dazu eingerichtet ist, bei einem Feststellen einer Abweichung von dem korrekten Betrieb der Ultraviolett-Bestrahlungseinheit (22) eine Benachrichtigung auszugeben.

7. Kompressor nach einem der vorhergehenden Ansprüche, wobei die Ultraviolett-Bestrahlungseinheit (22) innerhalb des Gehäuses (12) in einer vibrationsgedämpften Weise angebracht ist, insbesondere mittels wenigstens einer Federklemme (20a, 20b), welche vorzugsweise wenigstens abschnittsweise aus einem Keramik-und/oder Blechmaterial gefertigt ist.

8. Kompressor nach einem der vorhergehenden Ansprüche, ferner umfassend wenigstens eine an der Außenseite des Gehäuses (12) vorgesehene Halterung (34), welche vorzugsweise wenigstens ein Dämpfungselement (36) für mechanische Vibrationen umfasst, beispielsweise ein Elastomer-Dämpfungselement, wobei die Halterung (34) vorzugsweise wenigstens einen Permanentmagneten (38) umfasst.

9. Kompressor nach einem der vorhergehenden Ansprüche, wobei die Ultraviolett-Bestrahlungseinheit (22) dazu eingerichtet ist, ultraviolettes Licht im UVC-Bereich abzustrahlen, vorzugsweise bei einer Wellenlänge von etwa 254 nm.

10. Kompressor nach einem der vorhergehenden Ansprüche, wobei der Partikel-Vorfilter (16) wenigstens von der Filterklasse F7 ist.

11. Kompressor nach einem der vorhergehenden Ansprüche, ferner umfassend eine Unterdruck-Überwachungseinheit (18), welche dazu eingerichtet ist, über eine übermäßige Druckdifferenz zwischen dem Innenraum und der Umgebung des Gehäuses (12) zu benachrichtigen, und welche insbesondere im Bereich des Partikel-Vorfilters (16) angeordnet sein kann.

12. Kompressor nach einem der Ansprüche 5 bis 11, ferner umfassend eine Steuereinheit, welche mit der Überwachungseinheit betriebsmäßig gekoppelt oder integriert ist und dazu eingerichtet ist, bei einem Feststellen einer Abweichung von dem korrekten Betrieb der Ultraviolett-Bestrahlungseinheit (22) den Betrieb des Kompressors anzupassen, beispielsweise den Kompressor abzuschalten oder ein Spülventil zu öffnen.

13. Kompressor nach einem der vorhergehenden Ansprüche, ferner umfassend eine Zeitnahmeeinheit, welche dazu eingerichtet ist, einen zeitlichen Abstand zwischen einer Inbetriebnahme der Ultraviolett-Bestrahlungseinheit (22) und des wenigstens einen Kompressormotors sicherzustellen.

14. Kompressor nach einem der vorhergehenden Ansprüche, wobei ein Ansaugschlauch (30) zwischen der Filtereinheit (10) und dem Kompressorblock vorgesehen ist, welcher an dem Auslass (28) der Filtereinheit (10) vorzugsweise mittels elastischem Material abgedichtet angebracht ist.

15. Kompressor nach einem der vorhergehenden Ansprüche, , wobei die Sicherheitseinrichtung Mittel umfasst, durch welche ein Betrieb des Kompressors ausnahmsweise auch in Fällen erlaubt werden kann, in welchen die Filtereinheit nicht in Betrieb ist.

16. Verfahren zum Komprimieren von Atemluft mittels eines Kompressors nach einem der vorhergehenden Ansprüche, wobei die zu komprimierende Atemluft durch die Filtereinheit (10) angesaugt, anschießend durch den wenigstens einen Kompressormotor verdichtet und schließlich bei einem Druck von beispielsweise zwischen 90 und 550 bar abgefüllt wird.

## Claims

1. Compressor, in particular a breathing air compressor and/or multi-stage piston compressor, comprising a compressor block with at least one compressor motor and a filter unit (10), the filter unit (10) comprising:
- a housing (12) having an inlet (14) and an outlet (28) for gas to be compressed;
- a particle pre-filter (16) arranged downstream of the inlet (14) in the flow direction of the gas to be compressed;
- an ultraviolet irradiation unit (22) which is arranged downstream of the particle pre-filter (16) in the flow direction of the gas to be compressed and is configured to emit ultraviolet light; and
- a ballast (40) which is configured and arranged to control the ultraviolet irradiation unit (22);
wherein the filter unit (10) is designed in such a way that gas to be compressed which is fed in at the inlet (14) first passes through the particle pre-filter (16), is then irradiated by the ultraviolet irradiation unit (22) and can finally be fed to the compressor through the outlet (28),
**characterized in that**
the compressor further comprises a safety device which is configured to ensure that operation of the compressor is only possible when the filter unit is in operation, and which additionally comprises means by which operation of the compressor can exceptionally also be allowed in cases in which the filter unit is not in operation.

2. Compressor according to claim 1, in which the housing (12) in the area of the ultraviolet irradiation unit (22) is made at least partially, preferably completely, of anodised aluminium, in particular on the inside, wherein, for example, cartridge tubes, in particular with article numbers 61089, 62333 and 60174, can be used.

3. Compressor according to any of the preceding claims, wherein the housing (12) is elongate, in particular cylindrical, for example circular-cylindrical, in the region of the ultraviolet irradiation unit (22).

4. Compressor according to any of the preceding claims, wherein the ballast (40) is further configured to adapt the control of the ultraviolet irradiation unit (22) to an ambient temperature and/or a temperature of the gas to be compressed, which can be entered by a user using an input unit or can be detected by a sensor unit.

5. Compressor according to any of the preceding claims, further comprising a monitoring unit which is configured to monitor correct operation of the ultraviolet irradiation unit (22).

6. Compressor according to claim 5, further comprising a notification unit which is operatively coupled to the monitoring unit and is configured to output a notification when a deviation from the correct operation of the ultraviolet irradiation unit (22) is detected.

7. Compressor according to any of the preceding claims, wherein the ultraviolet irradiation unit (22) is fastened inside the housing (12) in a vibration-damped manner, in particular by means of at least one spring clip (20a, 20b), which is preferably made at least partially from a ceramic and/or sheet metal material.

8. Compressor according to any of the preceding claims, further comprising at least one bracket (34) which is provided on the outside of the housing (12) and preferably comprises at least one damping element (36) for mechanical vibrations, for example an elastomeric damping element, wherein the bracket (34) preferably comprises at least one permanent magnet (38).

9. Compressor according to any of the preceding claims, wherein the ultraviolet irradiation unit (22) is configured to emit ultraviolet light in the UVC range, preferably at a wavelength of about 254 nm.

10. Compressor according to any of the preceding claims, wherein the particle pre-filter (16) is at least of filter class F7.

11. Compressor according to any of the preceding claims, further comprising a vacuum monitoring unit (18) which is configured to provide a notification of an excessive pressure difference between the interior and the surroundings of the housing (12), and which can be arranged, in particular, in the region of the particle pre-filter (16).

12. Compressor according to any of claims 5 to 11, further comprising a control unit which is operatively coupled to or integrated with the monitoring unit and is configured to adapt the operation of the compressor, for example to switch off the compressor or to open a flush valve, when a deviation from the correct operation of the ultraviolet irradiation unit (22) is detected.

13. Compressor according to any of the preceding claims, further comprising a timing unit which is configured to ensure a time interval between a start-up of the ultraviolet irradiation unit (22) and of the at least one compressor motor.

14. Compressor according to any of claims 12 to 14, wherein a suction hose (30) which is attached, preferably sealed by means of elastic material, to the outlet (28) of the filter unit (10) is provided between the filter unit (10) and the compressor block.

15. Compressor according to any of the preceding claims, wherein the safety device comprises means by which operation of the compressor can exceptionally also be allowed in cases in which the filter unit is not in operation.

16. Method for compressing breathing air using a compressor according to any of the preceding claims, wherein the breathing air to be compressed is drawn in through the filter unit (10), then compressed by the at least one compressor motor and finally bottled at a pressure of, for example, between 90 and 550 bars.

## Revendications

1. Compresseur, en particulier compresseur d'air respirable et/ou compresseur à piston à plusieurs étages, comprenant un bloc de compresseur avec au moins un moteur de compresseur et une unité de filtration (10), l'unité de filtration (10) comprenant :
• un boîtier (12) avec une entrée (14) et une sortie (28) pour du gaz à comprimer ;
• un préfiltre à particules (16) disposé en aval de l'entrée (14) dans le sens d'écoulement du gaz à comprimer ;
• une unité d'irradiation ultraviolette (22) disposée en aval du préfiltre à particules (16) dans le sens d'écoulement du gaz à comprimer, laquelle est conçue pour émettre de la lumière ultraviolette ; et
• un régulateur de puissance (40) qui est conçu et disposé pour commander l'unité d'irradiation ultraviolette (22) ;
dans lequel l'unité de filtration (10) est conçue de telle sorte que le gaz à comprimer amené à l'entrée (14) traverse d'abord le préfiltre à particules (16), puis est irradié par l'unité d'irradiation ultraviolette (22) et peut enfin être acheminé vers le compresseur par la sortie (28),
**caractérisé en ce que**
le compresseur comprend en outre un dispositif de sécurité qui est conçu pour garantir que le compresseur ne peut fonctionner que lorsque l'unité de filtrage est en service, et qui comprend en outre des moyens permettant, à titre exceptionnel, de faire fonctionner le compresseur même lorsque l'unité de filtrage n'est pas en service.

2. Compresseur selon la revendication 1, dans lequel le boîtier (12) est fabriqué, au moins en partie, de préférence entièrement, en particulier sur sa face intérieure, en aluminium anodisé dans la zone de l'unité d'irradiation ultraviolette (22), dans lequel par exemple des cartouches peuvent être utilisées, en particulier celles portant les numéros d'article 61089, 62333 et 60174.

3. Compresseur selon l'une des revendications précédentes, dans lequel le boîtier (12) est de forme allongée, en particulier cylindrique, par exemple cylindrique circulaire, dans la zone de l'unité d'irradiation ultraviolette (22).

4. Compresseur selon l'une des revendications précédentes, le dispositif de commande (40) étant en outre conçu pour adapter la commande de l'unité d'irradiation ultraviolette (22) à une température ambiante et/ou à une température du gaz à comprimer, qui peut être saisie par un utilisateur à l'aide d'une unité de saisie ou détectée au moyen d'une unité de détection.

5. Compresseur selon l'une des revendications précédentes, comprenant en outre une unité de surveillance qui est conçue pour surveiller le bon fonctionnement de l'unité d'irradiation ultraviolette (22).

6. Compresseur selon la revendication 5, comprenant en outre une unité de notification qui est couplée de manière opérationnelle à l'unité de surveillance et qui est conçue pour émettre une notification lorsqu'un écart par rapport au bon fonctionnement de l'unité d'irradiation ultraviolette (22) est détecté.

7. Compresseur selon l'une des revendications précédentes, l'unité d'irradiation ultraviolette (22) étant montée à l'intérieur du boîtier (12) de manière à amortir les vibrations, en particulier au moyen d'au moins une pince à ressort (20a, 20b) qui est de préférence fabriquée au moins en partie à partir d'un matériau céramique et/ou métallique.

8. Compresseur selon l'une des revendications précédentes, comprenant en outre au moins un support (34) prévu sur la face extérieure du boîtier (12), qui comprend de préférence au moins un élément d'amortissement (36) pour les vibrations mécaniques, par exemple un élément d'amortissement en élastomère, le support (34) comprenant de préférence au moins un aimant permanent (38).

9. Compresseur selon l'une des revendications précédentes, dans lequel l'unité d'irradiation ultraviolette (22) est conçue pour émettre une lumière ultraviolette dans la gamme UVC, de préférence à une longueur d'onde d'environ 254 nm.

10. Compresseur selon l'une des revendications précédentes, dans lequel le préfiltre à particules (16) est au moins de classe de filtration F7.

11. Compresseur selon l'une des revendications précédentes, comprenant en outre une unité de surveillance de dépression (18) qui est conçue pour signaler une différence de pression excessive entre l'espace intérieur et l'environnement du boîtier (12) et qui peut être disposée en particulier dans la zone du préfiltre à particules (16).

12. Compresseur selon l'une des revendications 5 à 11, comprenant en outre une unité de commande qui est couplée de manière opérationnelle à l'unité de surveillance ou intégrée à celle-ci et qui est conçue pour adapter le fonctionnement du compresseur, par exemple pour arrêter le compresseur ou ouvrir une vanne de purge, lorsqu'elle détecte un écart par rapport au fonctionnement correct de l'unité d'irradiation ultraviolette (22).

13. Compresseur selon l'une des revendications précédentes, comprenant en outre une unité de chronométrage qui est conçue pour garantir un intervalle de temps entre la mise en service de l'unité d'irradiation ultraviolette (22) et dudit au moins un moteur de compresseur.

14. Compresseur selon l'une des revendications précédentes, dans lequel un tuyau d'aspiration (30) est prévu entre l'unité de filtration (10) et le bloc compresseur, lequel est fixé de manière étanche à la sortie (28) de l'unité de filtration (10), de préférence au moyen d'un matériau élastique.

15. Compresseur selon l'une des revendications précédentes, dans lequel le dispositif de sécurité comprend des moyens permettant, à titre exceptionnel, le fonctionnement du compresseur même lorsque l'unité de filtration n'est pas en service.

16. Procédé pour comprimer de l'air respirable à l'aide d'un compresseur selon l'une des revendications précédentes, dans lequel l'air respirable à comprimer est aspiré à travers l'unité de filtration (10), puis comprimé par ledit au moins un moteur de compresseur et enfin mis sous pression, avec une pression par exemple entre 90 et 550 bars.
